# EUROPÄISCHE PATENTSCHRIFT

(11) **EP 2 266 637 B1**
(45) Veröffentlichungstag und Bekanntmachung des Hinweises auf die Patenterteilung: **03.10.2018**
(21) Anmeldenummer: 10158589.1
(22) Anmeldetag: 31.03.2010
(51) Int. Cl.: A61L 31/02, A61L 31/08, A61L 31/14

(54) **Implantat und Verfahren zur Herstellung desselben**
Implant and method for production of the same
Implant et procédé pour sa préparation

(30) Priorität: 23.06.2009 US 219402 P
(43) Veröffentlichungstag der Anmeldung: 29.12.2010
(60) Teilanmeldung: 18190657.9
(73) Patentinhaber: BIOTRONIK AG, 8180 Bülach (CH)
(72) Erfinder: Klocke, Björn, 8006, Zürich (CH); Bayer, Ullrich, 18211, Admannshagen-Bargeshagen (DE)
(74) Vertreter: Keck, Hans-Georg

(56) Entgegenhaltungen:
- WO-A2-03/055537
- US-A1- 2006 229 711
- PEUSTER M ET AL: "Long-term biocompatibility of a corrodible peripheral iron stent in the porcine descending aorta", BIOMATERIALS, ELSEVIER SCIENCE PUBLISHERS BV., BARKING, GB, Bd. 27, Nr. 28, 1. Oktober 2006 (2006-10-01), Seiten 4955-4962, XP027951224, ISSN: 0142-9612 [gefunden am 2006-10-01]

## Beschreibung

Die vorliegende Erfindung betrifft ein Implantat, insbesondere eine intraluminale Endoprothese, mit einem Körper enthaltend metallisches Material, vorzugsweise Eisen, insbesondere eine Eisenlegierung, sowie Verfahren zur Herstellung eines solchen Implantats.

Medizinische Endoprothesen oder Implantate für die unterschiedlichsten Anwendungen sind in großer Vielfalt aus dem Stand der Technik bekannt. Als Implantate im Sinne der vorliegenden Erfindung sind endovaskuläre Prothesen oder sonstige Endoprothesen, beispielsweise Stents, Befestigungselemente für Knochen, beispielsweise Schrauben, Platten oder Nägel, chirurgisches Nahtmaterial, Befestigungselemente für künstliche Herzklappen, Darmklammern, Gefäßclips, Prothesen im Bereich des Hart- und Weichgewebes sowie Ankerelemente für Elektroden, insbesondere von Schrittmachern oder Defibrillatoren zu verstehen.

Heutzutage werden als Implantate besonders häufig Stents eingesetzt, die zur Behandlung von Stenosen (Gefäßverengungen) dienen. Sie weisen einen Körper in der Form eines ggf. durchbrochenen rohrförmigen oder hohlzylinderförmigen Grundgitters auf, das an beiden Längsenden offen ist. Das rohrförmige Grundgitter einer derartigen Endoprothese wird in das zu behandelnde Gefäß eingesetzt und dient dazu, das Gefäß zu stützen. Stents haben sich insbesondere zur Behandlung von Gefäßerkrankungen etabliert. Durch den Einsatz von Stents können verengte Bereiche in den Gefäßen erweitert werden, so dass ein Lumengewinn resultiert. Durch den Einsatz von Stents oder anderen Implantaten kann zwar ein für den Therapieerfolg primär notwendiger optimaler Gefäßquerschnitt erreicht werden, allerdings initiiert die dauerhafte Anwesenheit eines derartigen Fremdkörpers eine Kaskade von mikrobiologischen Prozessen, die zu einem allmählichen Zuwachsen des Stents und im schlimmsten Fall zu einem Gefäßverschluss führen können. Neben diesem Phänomen der Restenose haben permanente Implantate eine Reihe von weiteren Risiken: chronische Entzündungen, mangelndes Einwachsen, Spätthrombosen, erschwerte medizinische Reintervention, unkontrollierte Ermüdungsbrüche etc. Ein Ansatz zur Lösung dieser Probleme besteht darin, den Stent bzw. andere Implantate aus einem biodegradierbaren Werkstoff zu fertigen.

Unter Biodegradation werden chemische, hydrolytische, enzymatische und andere stoffwechselbedingte Abbauprozesse im lebenden Organismus verstanden, die vor allem durch die mit dem biodegradierbaren Werkstoff des Implantats in Kontakt gelangenden Körperflüssigkeiten verursacht werden und zu einer allmählichen Auflösung der den biodegradierbaren Werkstoff enthaltenden Strukturen des Implantats führen. Das Implantat verliert durch diesen Prozess zu einem bestimmten Zeitpunkt seine mechanische Integrität. Synonym zu dem Begriff Biodegradation wird häufig der Begriff Biokorrosion verwendet. Der Begriff Bioresorption umfasst die anschließende Resorption der Abbauprodukte durch den lebenden Organismus.

Für den Körper biodegradierbarer Implantate geeignete Werkstoffe können beispielsweise Polymere oder Metalle enthalten. Der Körper kann dabei aus mehreren dieser Werkstoffe bestehen. Gemeinsames Merkmal dieser Werkstoffe ist ihre Biodegradierbarkeit. Beispiele für geeignete polymere Verbindungen sind Polymere aus der Gruppe Cellulose, Kollagen, Albumin, Casein, Polysaccharide (PSAC), Polylactid (PLA), Poly-L-Lactid (PLLA), Polyglykol (PGA), Poly-D,L-Lactid-co-glycolid (PDLLA-PGA), Polyhydroxybuttersäure (PHB), Polyhydroxyvaleriansäure (PHV), Polyalkylcarbonate, Polyorthoester, Polyethylenterephtalat (PET), Polymalonsäure (PML), Polyanhydride, Polyphosphazene, Polyaminosäuren und deren Copolymere sowie Hyaluronsäure. Die Polymere können je nach den gewünschten Eigenschaften in Reinform, in derivatisierter Form, in Form von Blends oder als Copolymere vorliegen. Metallische biodegradierbare Werkstoffe basieren vorwiegend auf Legierungen von Magnesium und Eisen. Die vorliegende Erfindung bezieht sich vorzugsweise auf Implantate, deren biodegradierbarer Werkstoff zumindest teilweise ein Metall enthält, vorzugsweise Eisen, Mangan, Zink und/oder Wolfram, insbesondere eine Eisenbasislegierung (im Folgenden kurz: Eisenlegierung).

Bei der Realisierung biodegradierbarer Implantate wird angestrebt, die Degradierbarkeit entsprechend der angestrebten Therapie bzw. der Anwendung des jeweiligen Implantats (koronar, intracranial, renal etc.) zu steuern. Für viele therapeutische Anwendungen ist beispielsweise ein wichtiger Zielkorridor, dass das Implantat in einem Zeitraum von vier Wochen bis sechs Monaten seine Integrität verliert, weil nach dieser Zeit die Heilungsprozesse des Körpers in der Regel die mechanische Funktion des Gewebes wieder hergestellt haben. Hierbei wird unter Integrität, d. h. mechanischer Integrität, die Eigenschaft verstanden, dass das Implantat gegenüber dem undegradierten Implantat kaum mechanische Einbußen besitzt. Dies bedeutet, dass das Implantat mechanisch noch so stabil ist, dass zum Beispiel der Kollapsdruck lediglich geringfügig, d. h. höchstens auf 80% des Nominalwerts, abgefallen ist. Das Implantat kann somit bei vorhandener Integrität seiner Hauptfunktion, dem Aufhalten des Gefäßes, noch nachkommen. Alternativ kann die Integrität dadurch definiert werden, dass das Implantat mechanisch so stabil ist, dass es in seinem Belastungszustand in dem Gefäß kaum geometrischen Veränderungen unterworfen ist, beispielsweise nicht nennenswert zusammensackt, d. h. unter Belastung mindestens 80% des Dilatationsdurchmessers aufweist, oder im Fall eines Stents kaum angebrochene tragende Streben aufweist.

Implantate mit einer Eisenlegierung, insbesondere eisenhaltige Stents, sind besonders kostengünstig und einfach herstellbar. Beispielsweise für die Behandlung von Stenosen verlieren diese Implantate allerdings ihre mechanische Integrität bzw. Stützwirkung erst nach einem vergleichsweise langen Zeitraum, d. h. erst nach einer Verweildauer in dem behandelten Organismus von ca. 2 Jahren. Dies bedeutet, dass die Verweildauer von eisenhaltigen Implantaten für diese Anwendung zu lang ist. Für andere Anwendungen der eisenhaltigen Implantate, beispielsweise in der Orthopädie, gilt dies analog für eisenbasierte Implantate und für Implantate aus anderen Legierungen, wie beispielsweise einigen Magnesiumlegierungen (z. B. WE 43).

Im Stand der Technik werden bereits unterschiedliche Mechanismen der Degradationskontrolle von Implantaten beschrieben. Diese basieren beispielsweise auf anorganischen und organischen Schutzschichten oder deren Kombination, die dem humanen Korrosionsmilieu und den dort ablaufenden Korrosionsvorgängen einen Widerstand entgegensetzen. Bisher bekannte Lösungen zeichnen sich dadurch aus, dass Sperrschichtwirkungen erzielt werden, die auf einer räumlichen und möglichst defektfreien Trennung des Korrosionsmediums von dem metallischen Material beruhen. Diese führen dazu, dass die Degradationszeit verlängert wird. So wird der Degradationsschutz durch verschieden zusammengesetzte Schutzschichten und durch definierte geometrische Abstände (Diffusionsbarrieren) zwischen dem Korrosionsmedium und dem degradablen metallischen Grundkörpermaterial abgesichert. Andere Lösungen basieren auf Legierungsbestandteilen des biodegradierbaren Materials des Implantat-Körpers, welche den Korrosionsprozess durch Verschiebung der Lage in der elektrochemischen Spannungsreihe beeinflussen. Weitere Lösungen auf dem Gebiet der gesteuerten Degradation rufen durch das Aufbringen von physikalischen (z. B. lokale Querschnittsveränderungen) und/oder chemischen Veränderungen der Stent-Oberfläche (z. B. lokal chemisch unterschiedlich zusammengesetzte Multilayer) Sollbrucheffekte hervor. Mit den zuvor genannten Lösungen gelingt es jedoch bei eisenbasierten Implantaten meist nicht, die durch den Degradationsprozess eintretende Auflösung und die daraus resultierenden Stegbrüche in das geforderte Zeitfenster zu legen, da die genannten Lösungen im Wesentlichen eine Verlängerung der Verweildauer des Materials bewirken. Die Folge ist eine entweder zu spät einsetzende bzw. eine zu große Variabilität der Degradation des Implantats.

US 2006/0229711 A1 beschreibt oberflächenbehandelte Implantate. WO 03/055537 A2 beschreibt Implantate umfassend einen Magnesiumwerkstoff. In Biomaterials, 27, 4955, 2006 werden Eisenstents beschrieben.

Ein weiteres Problem im Zusammenhang mit Beschichtungen ergibt sich auch daraus, dass Stents oder andere Implantate üblicherweise zwei Zustände annehmen, nämlich einen komprimierten Zustand mit einem kleinen Durchmesser und einen expandierten Zustand mit einem größeren Durchmesser. Im komprimierten Zustand kann das Implantat mittels eines Katheters in das zu stützende Gefäß eingeführt und an der zu behandelnden Stelle positioniert werden. Am Ort der Behandlung wird das Implantat dann beispielsweise mittels eines Ballonkatheters dilatiert bzw. (bei Verwendung einer Formgedächtnislegierung als Implantatmaterial) beispielsweise durch Erwärmung über eine Sprungtemperatur in den expandierten Zustand überführt. Aufgrund dieser Durchmesseränderung ist der Körper des Implantats hierbei einer mechanischen Belastung ausgesetzt. Weitere mechanische Belastungen des Implantats können während der Herstellung oder bei der Bewegung des Implantats im oder mit dem Gefäß, in das das Implantat eingesetzt ist, auftreten. Bei den oben genannten Beschichtungen ergibt sich somit der Nachteil, dass die Beschichtung während der Verformung des Implantats reißt (z. B. aufgrund der Bildung von Mikrorissen) oder auch teilweise entfernt wird. Hierdurch kann eine unspezifizierte lokale Degradation verursacht werden. Außerdem sind das Einsetzen und die Geschwindigkeit der Degradation von der Größe und der Verteilung der durch die Verformung entstehenden Mikrorisse abhängig, die als Fehlstellen schlecht kontrollierbar sind. Dies führt zu einer starken Streuung in den Degradationszeiten.

Folglich besteht die Aufgabe der vorliegenden Erfindung darin, ein Implantat, das insbesondere metallisches Material aufweist, zu schaffen, das im gewünschten Zielkorridor, insbesondere in einem kürzeren Zeitraum, degradiert. Dabei soll die Degradation zu einem kontrollierbaren Zeitpunkt stattfinden und zusätzlich die Dilatation bzw. Verformung des Implantats ohne nennenswerten Einfluss auf das Degradationsverhalten bleiben. Entsprechend besteht die Aufgabe der Erfindung außerdem darin, ein Verfahren zur Herstellung eines solchen Implantats anzugeben, das einfach und kostengünstig durchführbar ist.

Die obige Aufgabenstellung wird gelöst durch ein Implantat, dessen Körper zumindest auf einem Teil seiner Oberfläche eine erste Schicht mit einer ionischen Verbindung (Salz) aufweist, die Ionen mindestens eines Halogens enthält.

Bei der vorliegenden Erfindung umfasst der Körper des Implantats mindestens einen Teil des Implantats, vorzugsweise den Hauptteil des Implantats, welcher die mechanische Integrität des Implantats bewirkt. Ferner muss die erste Schicht keine vollflächige Bedeckung des Implantatkörpers bilden, sondern kann Bereiche aufweisen, in denen die Oberfläche des Implantatkörpers nicht oder nicht vollständig abgedeckt ist.

Die erfindungsgemäß auf der Oberfläche des Implantatkörpers angeordnete erste Schicht mit der ionischen Verbindung hat die Aufgabe, in vivo Halogenidionen, vorzugsweise Chloridionen und/oder Bromidionen freizusetzen. Hierdurch wird an der Oberfläche des Implantatkörpers eine gegenüber physiologischen Bedingungen erhöhte Konzentration dieser Ionen erreicht. Diese erhöhte Konzentration der Halogenidionen, vorzugsweise der Chloridionen und/oder Bromidionen, begünstigt die Degradation des darunter liegenden Materials des Implantatkörpers. Hierbei können Konzentrationen beispielsweise der Chloridionen bis zur Sättigungsgrenze von NaCl in Wasser realisiert werden.

Die anwesenden Chlorid- und/oder Bromidionen oder anderen Halogenidionen begünstigen die Lochfraßkorrosion und die Spaltkorrosion, wobei letztere nach dem Auftreten von ersten Rissen beim Dilatieren bzw. beim Auftreten von ersten Ermüdungsrissen durch pulsative Beanspruchung des Implantats beispielsweise in einem Gefäß stattfindet. Bei diesen Korrosionsprozessen wird der Implantatkörper über verschiedene bekannte Mechanismen, insbesondere trotz Vorhandensein einer Passivierungsschicht, angegriffen. Als Passivierungsschicht wird die korrosionsmindernde, natürliche Oxidschicht auf der Oberfläche eines metallischen Materials bezeichnet.

Bei der sogenannten Lochfraßkorrosion wird der Sauerstoff der Passivierungsschicht durch die Halogenidionen, beispielsweise die Chlorid- und/oder Bromidionen, aus der Passivierungsschicht des Implantatkörpers verdrängt. Durch Anlagerung weiterer Chlorid- und/oder Bromidionen entsteht ein Bereich, welcher nicht mehr durch eine Oxidschicht geschützt ist. Dieser Bereich bildet einen idealen Angriffspunkt für die Korrosion. Bei der Spaltkorrosion werden in den Bereichen, in denen Risse in der Passivierungsschicht z. B. durch das Dilatieren oder andere mechanische Beanspruchung entstehen, Halogenidionen, beispielsweise die Chlorid- und/oder Bromidionen, angelagert. Hierdurch wird in den Rissbereichen die Korrosion ebenfalls begünstigt.

Ein weiterer Vorteil der erfindungsgemäß vorgesehenen ersten Schicht besteht darin, dass die Haftung ggf. darüber liegender weiterer Funktionsschichten z. B. mit einer pharmazeutisch aktiven Substanz oder anderen Materialien, vorzugsweise Polymermaterialien, verbessert wird.

In einem Ausführungsbeispiel der Erfindung können in der ersten Schicht mit der ionischen Verbindung Haftvermittler-, diffusionserlaubende oder degradierbare Zwischenschichten vorgesehen sein.

In einem besonders bevorzugten Ausführungsbeispiel ist die ionische Verbindung eine Verbindung aus der Gruppe enthaltend NaCl, CaCl₂ und MgCl₂. Diese Verbindungen lassen sich besonders kostengünstig herstellen bzw. sind einfach in der Handhabung.

Hierbei ist von Vorteil, wenn die ionischen Verbindungen trocken gelagert sind, da hierdurch die korrosionsbeschleunigende Wirkung der Salze durch die Zufuhr einer entsprechenden dissoziierenden Flüssigkeit, beispielsweise durch den Einbau des Implantats in einen Organismus und der Kontakt mit einer Körperflüssigkeit (Blut, Plasma) gesteuert werden kann.

Die ionische Verbindung kann in einem weiteren bevorzugten Ausführungsbeispiel mindestens ein Kation der Elemente aus der Gruppe enthaltend die Elemente der ersten Hauptgruppe, die Elemente der zweiten Hauptgruppe, Zink und Arsen aufweisen. Diese bilden zusammen mit den Chlorid- und/oder Bromidionen in der Produktion einfach handhabbare und kostengünstige ionische Verbindungen. Die in die erste Schicht eingebundene ionische Verbindung kann in einem bevorzugten Ausführungsbeispiel über das Kation auch eine positive Wirkung auf das umliegende Gewebe, z. B. eine antiproliferative Wirkung im Fall der Verwendung von Arsenchlorid als ionische Verbindung, haben.

In einem weiteren bevorzugten Ausführungsbeispiel weist die erste Schicht zusätzlich mindestens einen Carrier aus der Gruppe enthaltend Polylactide, Polyglycoside, Copolymere dieser Polymere und Erdalkaliphosphate auf und/oder es ist eine zweite Schicht vorgesehen, welche die erste Schicht mindestens teilweise bedeckt, wobei die zweite Schicht (Topcoat) eine Diffusionsbarriere für die Ionen des mindestens einen Halogens der ionischen Verbindung, insbesondere für die Chlorid- und/oder Bromidionen, bildet.

Der Carrier und/oder die zweite Schicht dienen dazu, dass die Chlorid- und/oder Bromidionen im Wesentlichen in der Nähe der Oberfläche des Implantatkörpers gehalten werden (insbesondere bei der Handhabung des Implantats) und nicht zu schnell in das umliegende Gewebe wegdiffundieren. Insbesondere durch die Aufbringung einer zweiten Schicht, welche vorzugsweise Polylactide, Polyglycoside, Copolymere dieser Polymere und Erdalkaliphosphate aufweist, wird der pH-Wert im Bereich der Oberfläche des Implantatkörpers verringert bzw. die Verringerung verstärkt oder, je nach Material des Implantatkörpers, überhaupt erst eine Korrosion ermöglicht. Im Falle der Verwendung eines Stents als Implantat wird in der Regel ein kompletter Abbau des Stentkörpers innerhalb weniger Monate erreicht.

In einem weiteren bevorzugten Ausführungsbeispiel ist zumindest auf einem Teil der ersten Schicht und gegebenenfalls der zweiten Schicht eine dritte Schicht vorgesehen, welche Magnesiumstearat und/oder Parylene und/oder eine pharmazeutische aktive Substanz aufweist. Im Falle der Anwesenheit eines Carriers kann die pharmazeutisch aktive Substanz auch direkt in die erste (bzw. zweite) Schicht eingebettet werden.

Unter einer "pharmazeutisch aktiven Substanz" (oder therapeutisch aktive oder wirksame Substanz) im Sinne der Erfindung wird ein pflanzlicher, tierischer oder synthetischer Wirkstoff (Medikament) oder ein Hormon verstanden, das in geeigneter Dosierung als Therapeutikum zur Beeinflussung von Zuständen oder Funktionen des Körpers, als Ersatz für natürlich vom menschlichen oder tierischen Körper erzeugte Wirkstoffe, wie Insulin, sowie zur Beseitigung oder zum Unschädlichmachen von Krankheitserregern, Tumoren, Krebszellen oder Körperfremdstoffen Einsatz findet. Die Freisetzung der Substanz in der Umgebung des Implantats hat einen positiven Effekt auf den Heilungsverlauf oder wirkt pathologischen Veränderungen des Gewebes in Folge des chirurgischen Eingriffs entgegen bzw. dient dem Unschädlichmachen von maladen Zellen in der Onkologie.

Derartige pharmazeutisch aktive Substanzen weisen beispielsweise eine antiinflammatorische und/oder antiproliferative und/oder spasmolytische Wirkung auf, wodurch beispielsweise Restenosen, Entzündungen oder (Gefäß-)Spasmen vermieden werden können. Derartige Substanzen können beispielsweise aus einer oder mehreren Substanzen der Wirkstoffgruppe der Calciumkanalblocker, der Lipidregulatoren (wie beispielsweise Fibrate), der Immunsuppressiva, der Calcineurininhibitoren (wie beispielsweise Tacrolimus), der Antiflogistika (wie beispielsweise Cortison oder Dichlofenac), der Antiinflammatorica (wie beispielsweise Imidazole), der Antiallergika, der Oligonucleotide (wie beispielsweise dODN), der Östrogene (wie beispielsweise Genistein), der Endothelbildner (wie beispielsweise Fibrin), der Steroide, der Proteine, der Hormone, der Insuline, der Zytostatika, der Peptide, der Vasodilatatoren (wie beispielsweise Sartane) und der antiproliferativ wirkenden Stoffe, der Taxole oder Taxane, hier vorzugsweise Paclitaxel oder Sirolimus, Everolimus, Biolimus A9, Deforolimus und ihre Derivate bzw. Prodrugs, bestehen.

Eine Beschichtung der mit der ersten Schicht und/oder gegebenenfalls der zweiten Schicht versehenen Oberfläche des Implantats mittels Parylene und/oder Magnesiumstearat ist von Vorteil, da die Oberflächeneigenschaften, beispielsweise der während eines Nachbehandlungsschritts bereits erreichte Korrosionsfortschritt, durch die darüber liegende dritte Schicht 'eingefroren' werden. Hierdurch können die Oberflächeneigenschaften, die sonst ggf. von der Lagerungs- oder Transportdauer des Implantats bis zum Einbringen in den zu behandelnden Organismus abhängen, und damit auch die Degradationsdauer reproduzierbar und definiert eingestellt werden. Dieser Effekt beruht auf der Wirkung als Diffusionsbarriere gegenüber der Permeation von Wassermolekülen und Halogenidionen, insbesondere Chlorid- und Bromidionen.

Parylene ist die Bezeichnung für eine spezielle Klasse vollständig linearer, teilkristalliner, unvernetzter aromatischer Polymere. Die verschiedenen Polymere besitzen unterschiedliche Eigenschaften und lassen sich in vier Grundtypen einteilen, nämlich Parylene C, Parylene D, Parylene N und Parylene F. Für die weitere Beschichtung nach der Oberflächenbehandlung mit einer ionischen Verbindung wird vorzugsweise Parylene C verwendet.

Vorteilhaft wirkt sich bei einer Beschichtung mit Parylene die hohe Spaltgängigkeit von Parylene aus, so dass auch komplizierte Geometrien oder nicht ebene Oberflächenstrukturen beschichtet werden. Die für Parylene, insbesondere Parylene C, charakteristischen Permeationseigenschaften für Wasser, chloridhaltige Lösungen und Wasserstoff sorgen in Verbindung mit der darunter liegenden mit einer ionischen Verbindung versehenen Oberfläche für ein besonders gut steuerbares Degradationsverhalten des Implantats. Zudem leistet die Parylene-Schicht einen zusätzlichen Beitrag zur Vermeidung bzw. Behinderung des Rissfortschritts bei mechanischer Belastung und verhindert partielle Schichtablösungen.

Hierbei liegen bevorzugte Schichtdicken der Parylene-Beschichtung zwischen etwa 0,5 µm und etwa 5,0 µm.

Mittels des erfindungsgemäßen Verfahrens kann im Falle der zusätzlichen Beschichtung mit Magnesiumstearat ein Implantat hergestellt werden, das sich durch Defektfreiheit der Körperoberfläche durch nachträgliches Versiegeln auszeichnet. Lokale Fehlstellen und/oder auf der Körperoberfläche des Implantats vorhandene Poren und andere nicht ebene Oberflächenstrukturen werden wirksam vor dem Kontakt mit korrosiv wirkenden Körperflüssigkeiten geschützt. Die hydrophobe Oberflächeneigenschaft und der geringe Kristallwassergehalt des Magnesiumstearats, der auch durch einen vorzugsweise durchgeführten, sich an das Aufbringen der Magnesiumstearat-Beschichtung anschließenden Trocknungsschritt erzeugt wird, bewirken während der sich anschließenden Lagerung und dem Transport des Implantats eine äußerst geringe Diffusion von Wasser zum Grundwerkstoff des Implantatkörpers. Ebenso wird das Loslösen von Partikeln mit geringer Bindungsneigung von der Oberfläche des Implantat-Körpers beim Dilatieren verhindert. Diese Partikel verbleiben in der zähen, hoch flexiblen Magnesiumstearatschicht. Hierdurch ergibt sich eine erhöhte Hämo- beziehungsweise Biokompatibilität.

Aufgrund der Magnesiumstearat-Beschichtung des Implantat-Körpers wird in vorteilhafter Weise bewirkt, dass der Reibungskoeffizient des Implantats sinkt. Hieraus folgt, dass beim Verschieben beispielsweise eines Stents als Implantat in einem Katheter geringere Kräfte aufgewendet werden müssen. Dadurch wird im Falle eines Stents eine genauere Stentfixation ermöglicht. Zudem werden das Crimpen und die spätere Freisetzung des Implantats an dem zu behandelnden Ort vereinfacht.

In einem bevorzugten Ausführungsbeispiel des erfindungsgemäßen Verfahrens wird die Magnesiumstearat-Beschichtung mittels Eintauchen in eine Lösung aufgebracht, wobei die Lösung Magnesiumstearat und ein Lösungsmittel, vorzugsweise Aceton und/oder Isopropanol, enthält und vorzugsweise eine Temperatur zwischen etwa 10°C und dem jeweiligen Siedepunkt des Lösungsmittel aufweist. Alternativ kann die Magnesiumstearatschicht auch derart aufgebracht werden, dass die genannte magnesiumstearathaltige Lösung auf den Körper des Implantats aufgesprüht wird (spray coating). Dabei wird das Teil in einer Kammer an einem dünnen Draht aufgehängt und mittels einem rotierenden Teller (Chargenhalter) allseitig besprüht.

In einem bevorzugten Ausführungsbeispiel kann die Effektivität des Tauchprozesses durch Anlegen eines Drucks erhöht werden, der kleiner als der Umgebungsdruck, vorzugsweise kleiner als etwa 90% des Umgebungsdrucks ist, d. h. des Luftdrucks an dem Ort, an dem der Tauchprozess durchgeführt wird. Der hierbei eintretende Entgasungseffekt führt zu einer schnellen Füllung der filigranen Oberflächenstruktur des Implantats mit Magnesiumstearat. Nach einer Verweildauer von einigen Minuten in der Lösung, vorzugsweise von mindestens etwa 2 Minuten, wird der mit Magnesiumstearat beschichtete Implantat-Körper aus dem Tauchbad entfernt und im Trockenofen bei einer Temperatur, die größer ist als die Raumtemperatur, vorzugsweise größer als etwa 30°C, getrocknet. Hierbei ist es besonders bevorzugt, wenn die Trocknungstemperatur möglichst gering ist, d. h. zwischen etwa 40°C und etwa 70°C liegt, da hierdurch ein langsames Freisetzen/Abdampfen des mindestens einen Lösungsmittels führt, wodurch einen porenfreie Schicht, die Magnesiumstearat enthält, erzeugt wird.

Die bevorzugte Dicke der Magnesiumstearat-Beschichtung liegt bei etwa 0,5 µm bis etwa 2,0 µm, vorzugsweise etwa 0,7 µm bis etwa 1,0 µm. Die Konzentration des Magnesiumstearats in der weiteren Beschichtung liegt dabei etwa zwischen 80 Gew.% und 100 Gew.%.

In einem bevorzugten Ausführungsbeispiel enthält der Körper des Implantats vorzugsweise ein degradierbares metallisches Material, vorzugsweise überwiegend Eisen, insbesondere mehr als 80 Gew.% Eisen, besonders bevorzugt mindestens 99 Gew.% Eisen, insbesondere in einer Legierung. Als weitere metallische Materialien können alternativ oder zusätzlich Mangan, Zink und/oder Wolfram eingesetzt werden.

Diese Implantate werden, da sie kostengünstig herstellbar sind, besonders gern für die Behandlung von Erkrankungen des menschlichen oder tierischen Organismus eingesetzt. Insbesondere bei eisenhaltigen Implantaten führt die Beschichtung der Oberfläche mit einer ersten Schicht, welche eine ionische Verbindung mit einem Halogenidion enthält, zu einer verringerten Degradationsdauer. Hierdurch wird eine Lücke zwischen den degradierbaren und nicht degradierbaren Legierungen für Implantate geschlossen.

Die obige Aufgabe wird ferner gelöst durch ein Verfahren umfassend die folgenden Schritte:
a) Bereitstellen des Körpers des Implants,
b) Aufbringen der Bestandteile der ersten Schicht mit mindestens einer ionischen Verbindung, die Ionen mindestens eines Halogens, vorzugsweise Chloridionen und/oder Bromidionen, aufweist, auf mindestens einen Teil der Oberfläche des Implantatkörpers (z. B. mittels nasschemischem Tauchen oder Sprühen und anschließendem Trocknen), wobei die ionische Verbindung vorzugsweise separat von weiteren Bestandteilen der ersten Schicht aufgebracht wird (z. B. durch Auftragen eines Polymers als Carrier in einem Gemisch mit einem Lösungsmittel oder durch Auftragen mittels eines plasmachemischen Verfahrens).

Ein derartiges Verfahren ist kostengünstig und erzeugt ein Implantat, welches die oben beschriebenen vorteilhaften Eigenschaften aufweist.

Das Aufbringen der ionischen Verbindung auf die Oberfläche des Implantatkörpers in Form einer Beschichtung ist von Vorteil, da hierdurch die Degradation auch räumlich bzw. örtlich gesteuert werden kann. In einigen Fällen ist es nämlich vorteilhaft, wenn zuerst eine Fragmentierung des Implantats, d. h. eine gezielte Korrosion des Implantatkörpers in bestimmten, vorgegebenen Bereichen, eingeleitet wird, welche dann eine wesentlich schnellere Degradation bewirkt als eine Korrosion des gesamten Implantatkörpers zur gleichen Zeit. Die Fragmentierung des Implantats erfolgt insbesondere dadurch beschleunigt, da das Gewebe bei der Fragmentierung mit weniger Metallionen, vorzugsweise Eisenionen, pro Zeiteinheit verglichen mit der Gesamtkorrosion belastet wird bzw. die Metallionen, vorzugsweise Eisenionen, in kleineren Mengen freigesetzt werden. Andererseits kann lokal die Korrosion schneller voranschreiten, wenn im Implantat nicht degradierende, kathodische Bereiche vorhanden sind. Diese begünstigen die Korrosion. Durch eine partielle Beschichtung an Stellen, die schnell degradieren sollen, kann außerdem die Geometrie der Fragmente vorbestimmt werden. Beispielsweise sind für die Anwendung in einem Organismus kurze Fragmente mit Abmessungen unterhalb von 200 µm deutlich unkritischer als große. Dies ist insbesondere dann bedeutsam, wenn Implantate beispielsweise Aufgrund einer Beschichtung mit einer pharmazeutisch aktiven Substanz bis zu dem gewünschten Degradationszeitpunkt nicht eingewachsen sind und Fragmente des Implantats in das behandelte Gefäß hineinragen oder weggeschwemmt werden können.

Insbesondere kann im Fall eines Stents die Beschichtung so aufgebracht werden, dass die Verbinder eines Stents möglichst schnell degradieren, um eine gute Biegeflexibilität zu erzielen. Ferner sollen Stentringe erst dann brechen, wenn die Stützwirkung nicht mehr erforderlich ist, z. B. nach 3 bis 6 Monaten. Zudem sollen kleinere Fragmente erst dann entstehen, wenn der Stent vollständig eingewachsen ist (z. B. dann, wenn der Stent ca. 4 Wochen ohne Medikamentbeschichtung oder ca. 6 Monate mit Medikamentenbeschichtung an der behandelten Stelle des Organismus angeordnet ist).

In einem bevorzugten Ausführungsbeispiel wird vor dem Aufbringen des ersten Bestandteils der ersten Schicht ein Haftvermittler, vorzugsweise ein Silan oder SiC, aufgebracht. Das Silan führt zur Bildung von dünnen SiOx-Schichten. Alternativ kann aber auch eine SiC-Beschichtung per PVD oder PE-CVD Verfahren aufgebracht werden.
Des Weiteren können Sputterverfahren zum Einsatz kommen, mit denen dünne (10-30 nm) TiO₂- oder ZnO-Schichten auf die Endoprothesenoberflächen aufgebracht werden.
Der Haftvermittler verbessert die Haftung bzw. Verbindung der ersten Schicht auf dem Körper des Implantats. Die Haftvermittlung beruht dabei sowohl auf chemischen Bindungskräften als auch auf dem Formschluss, der durch Mikrorauheiten der Beschichtung hervorgerufen wird.

Es ist ferner bevorzugt, wenn die ionische Verbindung der ersten Schicht mit den Halogenidionen, vorzugsweise den Chloridionen und/oder dem Bromidionen, mittels Tauchen und/oder Sprühen und/oder Bestäuben und/oder mittels Aufstrahlen von Strahlteilchen während tribochemischen Behandlung aufgebracht wird. Durch die angegebenen Verfahren kann die erste Schicht mit der mindestens einen ionischen Verbindung kostengünstig aufgebracht werden.

Beispielsweise kann zunächst eine poröse Carrierschicht, beispielsweise Calciumphosphat, aufgetragen werden. Anschließend wird der mit der Calciumphosphat-Carrierschicht versehene Implantatkörper in eine Lake mit der mindestens einen ionischen Verbindung getaucht und danach getrocknet.

Wenn ein Polymer-Carrier verwendet wird, so ist es hinsichtlich der Produktionskosten vorteilhaft, wenn durch eine geeignete Wahl von (biokompatiblen) Kationen und Lösungsmitteln dafür gesorgt wird, dass eine Mischung aus dem Polymer-Carrier und der ionischen Verbindung durch ein Tauch- oder Sprühverfahren aufgebracht werden kann.

Vorzugsweise wird bei Verwendung einer Kombination von Polymer-Carrier und Salz das Salz getrennt von dem Polymer aufgebracht wird. Beispielsweise wird zunächst durch Sprühen eine Polymer-Teilschicht, d. h. eine Teilschicht der ersten Schicht, beispielsweise mit einer Schichtdicke zwischen 1 µm bis 5 µm aufgebracht. Danach wird ein mikrokristallines Salz aufgestäubt, beispielsweise 100 bis 200 µg Salz. Hiermit ist die Auftragung der ersten Schicht abgeschlossen. Anschließend wird eine zweite Schicht aufgebracht, welche einen Polymer-Carrier aufweist mit einer Schichtdicke zwischen 5 µm und 20 µm.

Nach der Beschichtung des so hergestellten Implantats ist es von Vorteil, wenn das Implantat zur weiteren Lagerung luftdicht verpackt wird, beispielsweise durch Verwendung eines Schutzgases oder Trockenmittels. Hierdurch wird das Implantat trocken gelagert und der Korrosionsprozess schreitet während der Lagerung nicht unkontrolliert fort.

Bei einer Aufbringung der ionischen Verbindung der ersten Schicht mittels Aufstrahlen von Strahlteilchen während einer tribochemischen Behandlung wird die ionische Verbindung vorzugsweise als Reagenz in einer Hülle (Mikroverkapselung) aufgebracht, wobei das in der Hülle verkapselte Reagenz das jeweilige Strahlteilchen bildet. Die Mikroverkapselung wird beim Auftreffen auf die Oberfläche des Implantatkörpers größtenteils zerstört und das Reagenz freigesetzt. Das Reagenz adhäriert ggf. zusammen mit dem Material der Mikroverkapselung auf der Oberfläche des Implantatkörpers. Dieses Ausführungsbeispiel beruht auf der Verwendung mikroverkapselter Strahlteilchen, die in Richtung der Oberfläche des Implantats beschleunigt werden, wobei die Beschleunigung in Richtung der Implantatoberfläche unter unterschiedlichen Drücken, variablen Winkeln und Abständen erfolgt.

Alternativ kann die ionische Verbindung auch ohne Mikroverkapselung tribochemisch aufgetragen werden. Die aufgetragenen Salze adhärieren auf der Oberfläche des Implantat-körpers.

Beispielsweise ist auch ein ausschließlicher Einsatz von korrosiv wirksamen Salzen als Strahlteilchen möglich. Insbesondere wird trocken gelagertes MgCl₂ als körniges Salz definierter Körnung als Strahlmittel ohne Zusatz von weiteren abrasiv wirkenden Hartstoffen verwendet. Trocken gelagertes MgCl₂ sollte aus dem Grund verwendet werden, da sonst Verklumpungsgefahr durch starke Hygroskopie des Materials besteht.

MgCl₂-Teilchen bewirken aufgrund ihrer geringen Härte keine wesentliche mechanische Oberflächenveränderung, d. h. keine wesentliche Oberflächenaufrauung. Allerdings adhäriert ein großer Anteil der MgCl₂-Teilchen an der Oberfläche des Implantats. Bei anschließender Lagerung des so behandelten Implantats unter extrem trockener Atmosphäre und unter Luftabschluss wird eine Korrosion der Oberfläche zunächst verzögert. Erst zum Zeitpunkt der Implantation und dem Kontakt des Implantats, beispielsweise eines Stents, mit der Körperflüssigkeit beginnt das anhaftende MgCl₂ stark korrosiv zu wirken, so dass eine beschleunigte Degradation erfolgt.

Bei nicht ausschließlich trockener Lagerung verbleiben die MgCl₂-Partikel temporär bis zu ihrer Auflösung in der tribochemisch erzeugten Submikroaufrauung, beschleunigen die Korrosion bei Anwesenheit von Luftfeuchtigkeit und vergrößern somit die Abmessungen der Kavität der Submikroaufrauung.

Die auf der Oberfläche des Implantatkörpers adhärierten Salze bewirken eine beschleunigte Korrosion der Oberfläche, wobei die Korrosion auch abhängig von der anschließend an die tribochemische Behandlung durchgeführten Nachbehandlung des Implantats ist. Vorzugsweise werden während der tribochemischen Behandlung zusätzlich auch Strahlteilchen verwendet, welche ein Hartstoffmaterial enthalten und welche eine mechanische Veränderung, vorzugsweise eine Zerklüftung, der Oberfläche bewirken. Derartige Hartstoffmaterialien sind Verbindungen aus der Gruppe enthaltend Oxide, insbesondere Al₂O₃, SiO₂, ZrO₂, Karbide, insbesondere TiC, SiC, B₄C, Be₂C, Oxikarbide, Nitride, insbesondere TiN, c-BN, Si₃N₄, AlN und TiAlN, natürlicher oder synthetischer Diamant sowie Bor.

Alle tribochemisch aufgebrachten Strahlteilchen befinden sich nach Abschluss der tribochemischen Behandlung zumindest teilweise auf der Oberfläche des Implantatkörpers oder in einer geringen Tiefe im Implantatkörper.

Um eine effektive Zerstörung der Mikroverkapselung beim Auftreffen auf die Implantat-körper-Oberfläche zu erreichen, weist die Mikroverkapselung vorzugsweise mindestens eine thermo- und/oder fotolabile und/oder mechanisch labile Sollbruchstelle auf. Die thermo- und/oder fotolabile Wand der Mikroverkapselung ist vorzugsweise mittels Azofunktionen (-N=N-) oder Dioxyfunktionen (-O-O-) ausgebildet. Ferner können mechanische Sollbruchstellen in der Mikroverkapselung Aufgrund von Wanddickenunterschieden vorgesehen sein, die durch eine entsprechende geometrische Oberflächenstruktur der Mikroverkapselung realisiert sind. Hierbei stellen die dünnsten Bereiche der Mikroverkapselung die mechanischen Sollbruchstellen dar, welche beim Auftreffen der mikroverkapselten Strahlteilchen auf die Implantatoberfläche brechen und die gewünschte Freisetzung des innerhalb der Mikroverkapselung angeordneten chemisch aktiven Reagenz bewirken. Dabei wird beim Auftreffen der mikroverkapselten Strahlteilchen auf die Oberfläche des Implantats zunächst die Mikroverkapselung, welche beispielsweise aus einem Polymermaterial besteht, massiv plastisch verformt. Der überwiegende Teil der Mikroverkapselung wird hierbei zerstört, die Hülle platzt in den Bereichen mit der geringsten Wanddicke auf und das im Inneren angeordnete chemische Reagenz wird freigelegt. Der nicht sofort von der Implantatoberfläche abprallende bzw. reflektierte Teil der mikroverkapselten Strahlteilchen, d. h. Teile der Mikroverkapselung (Hülle) und/oder mindestens ein Teil des im Inneren enthaltenen Reagenz, adhäriert an der Oberfläche des Implantats. Hierdurch wird eine chemische Reaktion in Gang gesetzt, an der das Material des Implantats, das Material der Mikroverkapselung und das freigelegte chemische Reagenz beteiligt sind. Eine erhöhte Luftfeuchtigkeit und eine erhöhte Temperatur in der Behandlungskammer beschleunigen den durch die chemische Reaktion initiierten Korrosionsvorgang.

Bevorzugt wird ein bioabbaubares Polymer als Material der Mikroverkapselung und ein aktives Reagenz verwendet werden, welche zu Degradationseffekten an der Implantatoberfläche führt, die bei Einwirkung der einzelnen Komponenten so nicht zustande gekommen wäre. So bewirkt beispielsweise eine Reaktion zwischen einem Polymer wie PLA als Material der Mikroverkapselung und NaCl oder MgCl₂ als Reagenz eine starke Verschiebung des pH-Wertes an der Oberfläche des Implantats in den sauren Bereich. Die hierdurch vor allem im feuchten Milieu einsetzende heftige Korrosion führt zu einer Bildung von Eisenoxyhydroxiden. Diese haften nur schwach am Material des Implantats und werden im Laufe der fortschreitenden Korrosion durch die Chloridionen enthaltende Körperflüssigkeit (Plasma, Blut) unterwandert. Hierdurch wird eine starke Aufrauung der Oberfläche des Implantats bewirkt, so dass sich der Oberflächeninhalt des Implantats vergrößert und die Korrosion in die Tiefe fortschreitet. Diese Effekte stellen sich bereits nach sehr kurzen Zeiträumen von wenigen Sekunden bis Minuten ein und können die durch die während der laufenden tribochemischen Oberflächenbehandlung neu herangeführten Strahlteilchen erzeugte Korrosion des Implantatmaterials noch weiter verstärken. Insgesamt entsteht somit nach der tribochemischen Oberflächenbehandlung mittels mikroverkapselter Strahlteilchen eine aufgeraute Oberfläche, die sich aufgrund Ihrer Rauheit und Oberflächenaktivität für eine weitere Oberflächenbehandlung (z. B. finales Tauchen in biodegradierbares Polymer) sehr gut eignet.

In einem weiteren bevorzugten Ausführungsbeispiel erfolgt die tribochemische Behandlung in mindestens zwei Schritten, nämlich in einem ersten Schritt eine tribochemische Behandlung mittels Strahlteilchen, die ausschließlich oder zumindest überwiegend mindestens ein inertes Hartmaterial aufweisen, und in einem zweiten, sich an den ersten Schritt anschließenden Schritt eine tribochemischen Behandlung mittels Strahlteilchen, die zumindest teilweise das mindestens eine Salz, ggf. als Reagenz in einer Mikroverkapselung, aufweisen.

In einem weiteren bevorzugten Ausführungsbeispiel erfolgt nach einer tribochemischen Behandlung eine Auslagerung des tribochemisch behandelten Implantatkörpers bei erhöhter Luftfeuchtigkeit, insbesondere bei einer Luftfeuchtigkeit größer als 80%, und/oder bei erhöhter Temperatur, insbesondere bei einer Temperatur von mehr als 50°C.

Durch die angegebene Nachbehandlung des Implantatkörpers, insbesondere nach einer tribochemischen Behandlung durch Beschuss mit Strahlteilchen, die das mindestens eine Salz enthalten, wird eine besonders beschleunigte Korrosion der Körperoberfläche erreicht. Diese Nachbehandlung, insbesondere die Auslagerung bei erhöhter Luftfeuchtigkeit, bewirkt die Entstehung von ganz spezifischen, lokal qualitativ unterschiedlichen Lochkorrosionseffekten.

Bei diesem Ausführungsbeispiel des erfindungsgemäßen Verfahrens wird das Implantat außerdem bevorzugt nach einer variablen, jedoch vorgegebenen Einwirkzeit aus der Kammer mit erhöhter Luftfeuchtigkeit und/oder erhöhter Temperatur (beispielsweise nach 24 Stunden bis 72 Stunden bei einer relativen Luftfeuchtigkeit von 80% bis 98% bei einer Temperatur von etwa 20°C bis 90°C) entnommen und ggf. mit destilliertem Wasser intensiv gespült. Zur Vermeidung von weiteren, dann unkontrolliert ablaufenden Korrosionsprozessen wird das Implantat anschließend mit einem üblichen Korrosionsinhibitor, beispielsweise Magnesiumstearat und/oder Parylene (siehe oben), versehen und trocken gelagert. Alternativ kann das behandelte Implantat auch unter Schutzgas gelagert werden. Auf den Korrosionsinhibitor kann bei einer Lagerung unter Schutzgas gegebenenfalls
auch verzichtet werden.

In einem weiteren Ausführungsbeispiel beträgt die mittlere Teilchengröße der Strahlteilchen etwa 5 nm bis etwa 20 µm, vorzugsweise etwa 1 µm bis etwa 3 µm. Diese Teilchengröße hat sich besonders vorteilhaft bei typischen Geometrien von Implantaten, insbesondere Stents (Stegbreiten ca. 100 µm), erwiesen. Bei einer tribochemischen Behandlung mit Teilchen von maximal einigen µm treten keine plastischen Verformungen der Implantatstruktur auf. Bei robusteren orthopädischen Implantaten können Strahlteilchen mit einer Partikelgröße von bis zu 20 µm zur Anwendung kommen. Hierbei wird die Partikelgröße unter Anwendung eines Rasterelektronenmikroskops (bei Partikelgrößen kleiner oder gleich 3 µm) oder eines Lichtmikroskops (bei Partikelgrößen größer als 3 µm) ermittelt. Die elektronen- oder lichtmikroskopischen Aufnahmen werden vermessen und aus den Messwerten, beispielsweise durch Mittelwertbildung (arithmetisches Mittel), die mittlere Partikelgröße bestimmt. Die Strahlteilchen bestehen demnach nicht aus einzelnen Atomen, Ionen oder Verbindungen sondern jeweils aus einer Gruppe oder einem Gefüge von Atomen, Ionen- oder Molekülverbindungen. Die tribochemische Behandlung wird in der Regel bei Raumtemperatur, d. h. im kalten Zustand des Implantatkörpers, durchgeführt.

Tribochemische Behandlungen können mit bekannten Strahlanlagen einfach und kostengünstig bewerkstelligt werden. Um die tribochemische Behandlung der gesamten Oberfläche eines Implantats mit einer Öffnung oder einem umschlossenen Hohlraum, vorzugsweise eines Stents, zu gewährleisten, werden bei dem erfindungsgemäßen Verfahren Bestrahlungseinrichtungen eingesetzt, welche einen Dorn als Strahldüse verwenden. Ein solcher Dorn weist im Bereich seiner Strahldüsen einen Durchmesser auf, der deutlich geringer ist als der Durchmesser der Öffnung oder des Hohlraums des Implantats, in die der Dorn zur Bestrahlung eingeführt wird. Beispielsweise weist der Dorn einer Bestrahlungseinrichtung zur Bestrahlung der inneren Oberfläche eines Stents im Bereich seiner Düsen einen Außendurchmesser auf, der deutlich kleiner ist als der Innendurchmesser des Stents.

Alternativ kann die tribochemische Behandlung auch in elektrischen Wechselfeldern erfolgen. Dies trifft dann zu, wenn elektrisch leitfähige Partikel (Metalle oder Hartstoffpartikel wie z. B. TiN) als Strahlteilchen zum Einsatz kommen. Dabei wird der als Strahldüse fungierende Dorn und die die Endoprothese umgebende und ebenfalls als Partikelreservoir dienende gelochte Hülse an eine Wechselspannungsquelle angeschlossen. Als Gegenpol dient das Implantat. Bei dem durch Schließen des elektrischen Kontakts startenden tribochemischen Prozess werden die gegenüber der Endoprothese auf Potential liegenden Partikel aus dem Dorn und der Hülse heraus in Richtung Endoprothese beschleunigt.

Durch den einstellbaren Potentialunterschied entstehen unterschiedliche Beschleunigungseffekte, die wiederum zu unterschiedlich hohen kinetischen Energien der Partikel führen. Damit sind die Rauheit der Körperoberfläche und die Tiefenwirkung hinsichtlich Erhöhung der Versetzungsdichte einstellbar.

Die obige Aufgabenstellung wird ferner gelöst durch ein Implantat erhältlich durch eines der oben beschriebenen Herstellungsverfahren. Die durch die Beschichtung des Implantat-körpers entstehenden Oberflächenmorphologien und Oberflächenzusammensetzungen sind charakteristisch für die Behandlung und am fertig hergestellten Implantat erkennbar.

Das erfindungsgemäße Verfahren bzw. das erfindungsgemäße Implantat wird nachfolgend in Beispielen anhand von Figuren erläutert. Dabei bilden alle beschriebenen und/oder bildlich dargestellten Merkmale den Gegenstand der Erfindung, unabhängig von ihrer Zusammenfassung in den Ansprüchen oder deren Rückbezüge.

Es zeigen schematisch:
- Figur 1: einen Querschnitt einer aufgerauten Oberfläche eines ersten Ausführungsbeispiels eines erfindungsgemäßen Implantats nach 24 Stunden Lagerung in feuchter Atmosphäre (80% Luftfeuchtigkeit) bei 50°C,
- Figur 2: einen Querschnitt einer elektrisch unterstützten tribochemischen Beschichtungskammer und
- Figur 3: einen Querschnitt eines zweiten Ausführungsbeispiels eines erfindungsgemäßen Implantats.

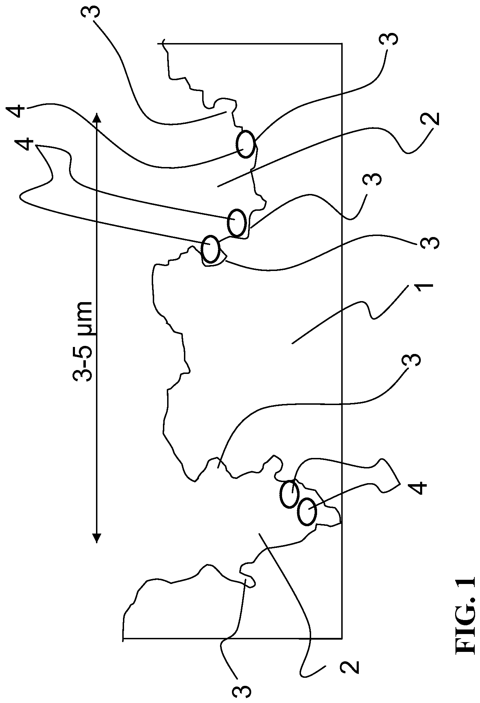

Figur 1 zeigt einen Querschnitt durch den oberflächennahen Bereich eines tribochemisch behandelten Implantats 1. Die Oberfläche weist Strukturen in Form von Mikroaufrauungen 2 auf, welche durch den Beschuss mit großen Hartstoff-Strahlteilchen und/oder Strahlteilchen höherer kinetischer Energie entstehen. Die Tiefe derartiger Mikroaufrauungen beträgt wenige Mikrometer. Die Größenverhältnisse sind durch den oben in der Figur 1 gezeichneten Pfeil dargestellt, welcher eine Abmessung von 3 µm bis 5 µm symbolisiert. Eine hohe kinetische Energie kann sowohl durch eine große Masse der Strahlteilchen als auch durch einen großen Strahldruck bzw. eine hohe Geschwindigkeit der Strahlteilchen erzielt werden. Ferner kann die kinetische Energie, wie oben dargestellt, durch die elektrischen Parameter des Wechselfelds variiert werden.

Die in dem Oberflächenprofil (der Oberflächenstruktur) enthaltenen Submikroaufrauungen 3, welche deutlich kleiner sind als die Mikroaufrauungen 2, entstehen durch den Einsatz kleiner salzhaltiger Strahlteilchen, welche an der Oberfläche als Salzteilchen 4 adhärieren und unter feuchten Umgebungsbedingungen sofort korrodierend wirken. Weiterhin können an der Entstehung von Submikroaufrauungen 3 auch die Korrosion begünstigende Reaktionen, initiiert durch das Aufstrahlen mikroverkapselter Strahlteilchen mit Sollbruchstellen in der Mikroverkapselung, beteiligt sein. Die Submikroaufrauungen 3 haben etwa einen Durchmesser von 0,1 µm bis 0,5 µm.

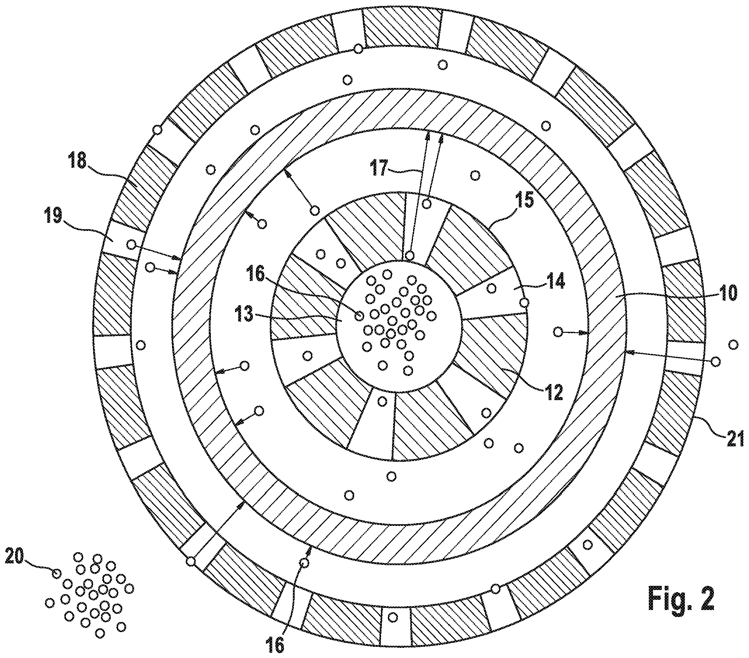

Figur 2 zeigt einen Querschnitt durch eine Anlage, mittels der die tribochemische Bestrahlung erfolgen kann. Beispielhaft wird durch die Anlage ein Stent 10 tribochemisch behandelt, welcher eine hohlzylindrische Form aufweist und somit im Querschnitt als Kreisring dargestellt ist. Im Innenvolumen des Stents 10 ist ein Dorn 12 mit einer an der Dornspitze vorgesehenen mittigen Öffnung 13 sowie radial verlaufenden Durchtrittsöffnungen 14 angeordnet. Ferner umgibt den Stent 10 eine Hülse 18 mit radial verlaufenden Durchtrittsöffnungen 19. In dem Dorn 12 und außerhalb der Hülse 18 ist ein Reservoir von Strahlteilchen 16 vorgesehen, welche von dem Dorn 12 zu Stent 10 bzw. von der Hülse 18 zu der Oberfläche des Stents 10 beschleunigt werden. Hierbei bewegen sich die Strahlteilchen 16 durch die entsprechenden Durchtrittsöffnungen 14 bzw. 19 hindurch. Die Bewegungsrichtung der Strahlteilchen 16 ist für einige Strahlteilchen durch einen Pfeil 17 angedeutet. Das Strahlteilchenreservoir der Hülse 18 ist in Figur 2 lediglich schematisch dargestellt und mit dem Bezugszeichen 20 versehen. Eine Erhöhung der kinetischen Energie der Strahlteilchen kann durch das Anlegen eines elektrischen Feldes erfolgen. Hierfür liegen einerseits der Stent 10 und anderseits der Dorn 12 bzw. die Hülse 18 auf einem unterschiedlichen elektrischen Potential. Zur Erzeugung der Potentialdifferenz weisen der Dorn 12 eine Polung 15 und die Hülse 18 eine Polung 21 auf.

### 1. Beispiel (tribochemische Behandlung eines Implantats mittels Hartstoff-Strahlteilchen)

Ein Stent aus einer Eisenbasislegierung mit einem Körper der Zusammensetzung 99,5 Gew.% Fe, weitere Elemente Mn, C, Si, P und S wird zunächst gemäß den üblichen Herstellungsverfahren (Laserschneiden des Rohres (Außendurchmesser 2,0 mm, Wanddicke 125 µm), Entgraten mittels Reibahle, Elektropolieren zum Zwecke der Kantenverrundung und der Entfernung der oxidischen Oberfläche) hergestellt. Alternativ kann auch ein C60-Stahl (Stahl mit 0,60 Gew.% Kohlenstoff, Rest Eisen) verwendet werden. Als weitere Alternative ist der Einsatz einer Eisenbasislegierung mit 12 Gew.% bis 20 Gew.% Mn denkbar.

Anschließend erfolgt die tribochemische Behandlung. Dazu wird die Stentoberfläche in einer Strahlkammer gleichzeitig innen und außen bei einem Strahldruck von 5 bar mit TiC Partikeln tribochemisch behandelt. Die mittlere Partikelgröße beträgt dabei 2 µm. Nach einem Zeitraum von 3 bis 5 Minuten wird dieser Teilprozess beendet. Der Stent wird der Behandlungskammer entnommen und in Ethanol zwischen gereinigt. Danach erfolgt die Rückführung in die gleiche Strahlkammer in der nun ein zweiter Verfahrenschritt erfolgt. In dem werden mit einem Polymer aus Polylactid (PLA) umhüllte Salzpartikel aus MgCl₂ auf die Oberfläche gestrahlt. Diese Partikel weisen einen Außendurchmesser von ca. 5 µm auf. Die sich im Inneren befindlichen MgCl₂ Partikel sind unregelmäßig geformt und haben eine maximale Ausdehnung von ca. 3 µm. Bei einem angelegten Strahldruck von ebenfalls ca. 4 bar zerplatzen diese beim Auftreffen auf die Stentoberfläche. Die sich dabei bildenden PLA - Fragmente und die dann frei gelegten MgCl₂ - Partikel adhärieren zum großen Teil an der vorher mit Hilfe der TiC-Partikel aufgerauten Oberfläche. Das heißt, die Oberfläche ist bei einer Behandlungszeit von 5 bis 10 min und in Abhängigkeit der auf die Oberfläche gestrahlten Partikelmenge mit einer bis zu 5 µm dicken Schicht belegt, die aus Bruchstücken der Polymerhülle und des freigelegten MgCl₂ besteht.

Optional besteht die Möglichkeit, die Adhäsion weiter zu verbessern, indem der Stent vor der tribochemischen Behandlung mit einem Sol-Gel-Verfahren behandelt (Tauchverfahren) und anschließend getempert wird. Hierdurch wird eine bis zu 20 nm dicke Haftvermittlerschicht aus SiOx erzeugt. Diese SiOx- Schicht bewirkt eine höhere Haftung der Strahlpartikel bzw. deren Fragmente an der Stentoberfläche. Alternativ zu einer SiOx-Schicht kann auch mittels PVD oder PE-CVD eine SiC-Schicht als Haftvermittler aufgetragen werden.

Die so erhaltene Oberfläche des Stents zeigt sowohl bei hoher Luftfeuchtigkeit als auch in vivo eine erhöhte Korrosionsneigung. Das PLA hydrolisiert und wird in vivo im weiteren Verlauf verstoffwechselt. Das MgCl₂ beschleunigt die Eisenkorrosion. Es kommt zur verstärkten Bildung von Eisenhydroxid und Eisenoxyhydroxid an der Stentoberfläche. Dieser Rost zeigt erwartungsgemäß auch hier nur eine lose Bindung zum Grundwerkstoff. Lose Korrosionsprodukte werden abgerissen, so dass immer wieder metallische Oberflächen freigelegt werden. Im Vergleich zu einem unbehandelten Eisenbasisstent mit identischer Zusammensetzung ist der korrosionsbedingte Masseverlust in vivo in einem Vergleichszeitraum zwischen 50% und 100% höher.

Das 1. Beispiel betrifft eine tribochemische Behandlung eines Implantats mittels Hartpartikeln. Die Hartpartikel bestehen aus einer Mischung aus mikroskaligen Strahlteilchen von TiC, WC und TiCN. Die mittlere Korngröße der Strahlteilchen beträgt 4 µm bei einer Streuung von +/- 1 µm. Die tribochemische Behandlung findet in einer tribochemischen Anlage statt. Mittels Druckluft bei 3 bis 4 bar Druck werden die Strahlteilchen mittels der in Figur 2 gezeigten Strahlanlage gleichzeitig auf die Innen- und die Außenseite des Implantates gestrahlt. Der Prozess erfolgt über einen Zeitraum von 5 Minuten. Infolgedessen kommt es zu elastischen und plastischen Verformungseffekten der Oberfläche des Implantats. Der plastische Verformungsanteil hinterlässt die in Figur 1 dargestellte Oberflächenmorphologie (ohne die durch nachträgliche Salze entstandenen Submikroaufrauungen).

Mikrohärteuntersuchungen eines derart behandelten Implantats aus Reineisen haben gezeigt, dass in dem durch die Strahlteilchen plastisch verformten oberflächennahen Volumenbereich eine im Vergleich zum Bauteilinneren um bis zu 150 HV 0,1 erhöhte Mikrohärte in einer Rautiefe von 10 µm vorliegt.

### 2. Beispiel (tribochemische Behandlung eines Implantats mittels Strahlteilchen aus Salzen)

Die Salze bestehen aus einer Mischung von jeweils 50 Masse% NaCl und MgCl₂. Die Teilchengröße der verwendeten Salze kann bei diesem Ausführungsbeispiel in einem breiten Bereich variieren (2 µm bis 200µm), da die Salzpartikel beim Auftreffen auf die Stentoberfläche zu kleineren Bruchstücken fragmentieren. Diese werden nach dem im Ausführungsbeispiel 1 beschriebenen Prozess auf die durch die Hartstoffpartikel bereits vorgeschädigte Oberfläche gestrahlt. Der Prozess wird in einer in Figur 2 im Schnitt dargestellten tribochemischen Behandlungsanlage bei den im Beispiel 1 genannten Drücken durchgeführt. Um ein Verklumpen der hygroskopischen Salzmischung zu vermeiden, muss diese vor und während des tribochemischen Behandlungsprozesses trocken gelagert werden.

Zusätzlich oder alternativ zur Beaufschlagung mit Druckluft kann die Hülse und der Dorn gegenüber dem Implantat mit einem von diesem verschiedenen elektrischen Potential beaufschlagt werden. Dies führt zu einer elektrischen Aufladung der Salzpartikel. Aus dieser resultiert eine - im Vergleich zur stromlosen Behandlungsvariante - erhöhte Beschleunigung der Partikel in Richtung Implantatoberfläche. Eine erhöhte temporäre Anhaftung der Salze in den Mikroaufrauungen und die daraus resultierende stärkere Ausprägung von Submikroaufrauungen sind die Folge.

### 3. Beispiel (wie eines der Beispiele 1. bis 2. und zusätzliche weitere Beschichtung mittels Parylene, Magnesiumstearat und/oder pharmazeutisch aktiver Substanz)

Auf der Basis der bisher genannten Ausführungsbeispiele kann als finaler Behandlungsschritt eine Beschichtung mit oben angegebenen Materialien erfolgen. Die Beschichtung mit Parylene C erfolgt aus der Gasphase. Nach ca. einer halben Stunde Beschichtungszeit wird eine Schichtdicke von ca. 0,5 µm erzielt.

Die Parylene-Beschichtung verfolgt dabei das Ziel eines temporären Korrosionsschutzes. Der "vorgeschädigte" Oberflächenzustand wird "eingefroren". Es erfolgt somit keine unkontrollierte selbstständig verlaufende Degradation vor dem Verbringen der Endoprothese an den Einsatzort.

Das gleiche Ziel wird mit der nachfolgend beschriebenen Magnesiumstearatbeschichtung verfolgt. Nach der Durchführung der Ausführungsbeispiele 1 und 2 und einer daran anschließenden Trocknung, wird die Endoprothese auf einen Kunststofffaden (z. B. Polyamid) aufgehängt und anschließend in die Lösung zur Auftragung des Magnesiumstearats getaucht. Die Lösung besteht z. B. aus 9 Teilen hochreinem Aceton oder Isopropanol und 1 Teil Magnesiumstearat. Der Tauchvorgang erfolgt bei Raumtemperatur in einem evakuierbaren Exsikkator. In diesem wird ein Unterdruck von ca. 100 mbar mittels einer Pumpe erzeugt. Hierdurch werden die filigranen und durch die vorangegangene plasmachemische Vorbehandlung entstandenen mikroporösen Oberflächenstrukturen bzw. die Hinterschneidungen und kompliziert geformten Strukturen effektiv von Restgas befreit. Dadurch kann in der Lösung eine vollständige Bedeckung der Stentoberfläche durch das Magnesiumstearat erfolgen, das auch in die Oberflächenstrukturen und Hinterschneidungen eindringt. Nach einer Verweildauer von etwa 3 Minuten in dem Tauchbad wird der Exsikkator belüftet, das Implantat aus dem Tauchbad entnommen und anschließend in einem Umluftschrank, immer noch am Kunststofffaden hängend, bei einer Temperatur von 60°C getrocknet. Die Schichtdicke der so erhaltenen Magnesiumstearat-Beschichtung liegt dabei im Bereich von 0,5 bis 10 µm.

Bedingt durch den im Exsikkator vorliegenden Unterdruck wird das Magnesiumstearat sehr gleichmäßig auf der Oberfläche abgeschieden. Eine geringe Trocknungstemperatur bewirkt in vorteilhafter Weise ein langsames Freisetzen/Abdampfen der Lösungsmittel der Tauch-Lösung, so dass eine porenfreie Magnesiumstearatschicht entsteht. Handelt es sich bei dem so hergestellten Implantat um einen Stent, so kann der mit der ersten Schicht und der Zwischenschicht versehene Körper anschließend mit einem Katheter komplettiert und einer Strahlensterilisation unterzogen werden.

Analog zu der Herstellung der Parylene- oder Magnesiumstearat-Beschichtung kann die Oberfläche des Implantats alternativ oder zusätzlich mit einer pharmazeutisch aktiven Substanz beschichtet werden. Bevorzugte Substanzen sind oben in der Beschreibung angegeben.

### 4. Beispiel (tribochemische Behandlung mit Hartstoffpartikeln und nachgeschaltetem Aufbringen einer haftvermittelnden Schicht für die Salze und anschließender Parylene - oder Magnesiumstearatbeschichtung)

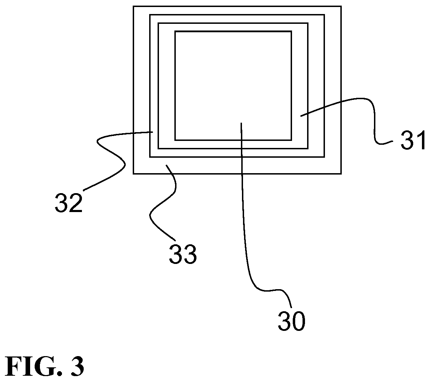

In Figur 3 ist ein Querschnitt eines Stentstegs gezeigt, welcher einen Körper 30 bestehend aus einer Eisenbasislegierung aufweist. Die Herstellung dieses Stents gestaltete sich folgendermaßen:
Auf der Oberfläche des Körpers 30 mit einem Querschnitt von ca. 100 µm x 100 µm ist eine erste Schicht 31 mit haftvermittelnden Eigenschaften existent. Diese besteht aus 10 bis 30 nm dicken Schichten aus SiOx oder SiC oder TiO₂ oder ZnO. Diese Haftvermittlerschicht wird auf die vorher mit Hartstoffpartikeln tribochemisch behandelte Oberfläche des Körpers 30 aufgetragen. Diese Oberflächenbehandlung mit inerten Hartstoffen führt nur zu einer Oberflächenverfestigung und einer damit verbundenen Erhöhung der Defektdichte in den oberflächennahen Randbereichen. Da es zu keinem Stoffschluss mit diesen Partikeln kommt, ist diese auch nicht Bestandteil des Querschnitts. Die zum Beispiel aus TiO₂ bestehende Schicht 31 wird mit einem bekannten Sputterverfahren oder einem Hochratezerstäuben erzeugt. Beide Verfahren erfolgen unter Vakuumbedingungen. Ein während des Prozesses in der Behandlungskammer zu realisierender Sauerstoffpartialdruck und das als Targetmaterial in der Beschichtungskammer vorliegende Titan führen unter den Bedingungen der physikalischen Dampfphasenabscheidung (PVD) zur Bildung von dünnen TiO₂ Schichten auf der Stentoberfläche.
Analog gilt dies auch für die anderen oxidischen Haftvermittlerschichten. Auf der ersten Schicht 31 liegt eine zweite Schicht 32 aus adhärierten Salzen aus 50 Masse% MgCl₂ und 50Masse% NaCl, die eine Schichtdicke von maximal 5 µm aufweist. Auf dieser befindet sich eine dritte Schicht 33 aus bis zu 10 µm Magnesiumstearat. Die Herstellung wurde realisiert wie in Ausführungsbeispiel 3 beschrieben. Die Schichten 31, 32 und 33 weisen eine Gesamtschichtdicke im Bereich von 1 µm bis 15 µm auf.

### Bezugszeichenliste:

- 1: Implantatkörper
- 2: Mikroaufrauung
- 3: Submikroaufrauung
- 4: adhäriertes Salzteilchen
- 10: Stent
- 12: Dorn
- 13: Öffnung
- 14: Durchtrittsöffnung für Strahlteilchen 16
- 15: Polung des Dorns
- 16: Strahlteilchen
- 17: Pfeil (Bewegungsrichtung des jeweiligen Strahlteilchens 16)
- 18: Hülse
- 19: Durchtrittsöffnung für Strahlteilchen 16
- 20: Strahlteilchenreservoir der Hülse 18
- 21: Polung der Hülse 18
- 30: Implantatkörper
- 31: erste Schicht
- 32: zweite Schicht
- 33: dritte Schicht

## Patentansprüche

1. Implantat, insbesondere eine intraluminalen Endoprothese, mit einem Körper (30) enthaltend metallisches Material, vorzugsweise Eisen, wobei der Implantatkörper (30) zumindest auf einem Teil seiner Oberfläche eine erste Schicht (31) mit mindestens einer ionischen Verbindung aufweist, die Ionen mindestens eines Halogens enthält, **dadurch gekennzeichnet, dass** zumindest auf einem Teil der ersten Schicht (31) oder ggf. einer zweiten Schicht (32), welche die erste Schicht mindestens teilweise bedeckt, wobei die zweite Schicht eine Diffusionsbarriere für die Ionen des mindestens einen Halogens der ionischen Verbindung bildet, eine dritte Schicht (33) angeordnet ist, welche Magnesiumstearat aufweist.

2. Implantat nach Anspruch 1, **dadurch gekennzeichnet, dass** die ionische Verbindung Chloridionen und/oder Bromidionen enthält.

3. Implantat nach einem der vorhergehenden Ansprüche, **dadurch gekennzeichnet, dass** die ionische Verbindung eine Verbindung aus der Gruppe enthaltend NaCl, CaCl₂, MgCl₂ ist.

4. Implantat nach einem der vorhergehenden Ansprüche, **dadurch gekennzeichnet, dass** die erste Schicht (31) zusätzlich mindestens einen Carrier aus der Gruppe enthaltend Polylactide, Polyglycoside, Copolymere dieser Polymere und Erdalkaliphosphate aufweist.

5. Implantat nach einem der vorhergehenden Ansprüche, **dadurch gekennzeichnet, dass** die ionische Verbindung mindestens ein Kation der Elemente aus der Gruppe enthaltend die Elemente der ersten Hauptgruppe, die Elemente der zweiten Hauptgruppe, Zink und Arsen aufweist.

6. Implantat nach einem der vorhergehenden Ansprüche, **dadurch gekennzeichnet, dass** zumindest auf einem Teil der ersten Schicht (31) oder ggf. der zweiten Schicht (32) eine dritte Schicht (33) angeordnet ist, welche Parylene und/oder eine pharmazeutisch aktive Substanz aufweist.

7. Implantat nach einem der vorhergehenden Ansprüche, **dadurch gekennzeichnet, dass** der Körper des Implantats überwiegend Eisen aufweist, vorzugsweise mehr als 80 Gew.%, besonders bevorzugt mehr als 99 Gew.% Eisen, insbesondere in einer Legierung, enthält.

8. Verfahren zur Herstellung eines Implantats, insbesondere nach einem der vorhergehenden Ansprüche, mit einem Körper (30) enthaltend metallisches Material, vorzugsweise Eisen, umfassend die folgenden Schritte:
a) Bereitstellen des Körpers des Implantats,
b) Aufbringen der Bestandteile der ersten Schicht (31) auf mindestens einen Teil der Oberfläche des Implantatkörpers, wobei vorzugsweise die ionische Verbindung separat von weiteren Bestandteilen der ersten Schicht (31) aufgebracht wird
c) Aufbringen auf zumindest einem Teil der ersten Schicht einer dritten Schicht, die Magnesiumstearat enthält.

9. Verfahren nach Anspruch 8, **dadurch gekennzeichnet, dass** vor dem Aufbringen des ersten Bestandteils der ersten Schicht (31) ein Haftvermittler auf die Oberfläche des Implantatkörpers zumindest in den Bereichen aufgebracht wird, auf die danach die Bestandteile der ersten Schicht angeordnet werden.

10. Verfahren nach Anspruch 8 oder 9, **dadurch gekennzeichnet, dass** zumindest die ionische Verbindung der ersten Schicht (31) mittels Tauchen und/oder Sprühen und/oder Bestäuben und/oder mittels Aufstrahlen von Strahlteilchen während einer tribochemischen Behandlung aufgebracht wird.

11. Verfahren nach Anspruch 10, **dadurch gekennzeichnet, dass** die Strahlteilchen die ionische Verbindung in einer Mikroverkapselung enthalten, wobei die Mikroverkapselung vorzugsweise mindestens eine thermo- und/oder eine photolabile Sollbruchstelle und/oder eine mechanisch labile Sollbruchstelle aufweist.

12. Verfahren nach Anspruch 10 oder 11, **dadurch gekennzeichnet, dass** die tribochemische Behandlung in mindestens zwei Schritten erfolgt, nämlich in einem ersten Schritt eine tribochemische Behandlung mittels Strahlteilchen, die ausschließlich oder zumindest überwiegend mindestens ein inertes Hartmaterial aufweisen, und in einem zweiten, auf den ersten Schritt folgenden Schritt eine tribochemische Behandlung mittels Strahlteilchen, welche die ionische Verbindung enthalten.

13. Verfahren nach einem der Ansprüche 10 bis 12, **dadurch gekennzeichnet, dass** nach einer tribochemischen Behandlung eine Auslagerung des tribochemisch behandelten Implantatkörpers bei erhöhter Luftfeuchtigkeit, insbesondere bei einer Luftfeuchtigkeit größer als 80%, und/oder bei erhöhter Temperatur, insbesondere bei einer Temperatur von mehr als 50°C, erfolgt.

14. Implantat, insbesondere intraluminale Endoprothese, mit einem Körper enthaltend metallisches Material, vorzugsweise Eisen, erhältlich durch ein Verfahren gemäß einem der Ansprüche 8 bis 13.

## Claims

1. Implant, particularly an intraluminal endoprosthesis, having a body (30) containing metallic material, preferably iron, **wherein** the implant body (30) has a first layer (31) on at least part of its surface, with at least one ionic compound that contains ions of at least one halogen, **characterized in that** a third layer (33) is provided which contains magnesium stearate at least on a part of the first layer (31) or eventually of a second layer (32), which covers the first layer at least in part, where the second layer forms a diffusion barrier for the ions of the at least one halogen of the ionic compound.

2. Implant according to claim 1, **characterized in that** the ionic compound contains chloride ions and/or bromide ions.

3. Implant according to one of the preceding claims, **characterized in that** the ionic compound is a compound from the group that contains NaCl, CaCl₂, MgCl₂.

4. Implant according to one of the preceding claims, **characterized in that** the first layer (31) additionally has at least one carrier from the group that contains polylactides, polyglycosides, copolymers of these polymers and earth alkali phosphates.

5. Implant according to one of the preceding claims, **characterized in that** the ionic compound has at least one cation of the elements from the group that contains the elements of the first main group, the elements of the second main group, zinc and arsenic.

6. Implant according to one of the preceding claims, **characterized in that** a third layer (33) is disposed at least on a part of the first layer (31) or if applicable the second layer (32), which third layer contains parylene and/or a pharmaceutically active substance.

7. Implant according to one of the preceding claims, **characterized in that** the body of the implant predominantly contains iron, preferably more than 80 wt.-%, particularly preferably more than 99 wt.-% iron, particularly in an alloy.

8. Method for the production of an implant, particularly according to one of the preceding claims, having a body (30) that contains metallic material, preferably iron, comprising the following steps:
a) making available the body of the implant,
b) applying the components of the first layer (31) to at least a part of the surface of the implant body, where preferably, the ionic compound is applied separately from other components of the first layer (31)
c) applying on at least a part of the first layer a third layer which contains magnesium stearate.

9. Method according to claim 8, **characterized in that** before application of the first component of the first layer (31), an adhesion-promoting agent is applied to the surface of the implant body, at least in the regions on which the components of the first layer are subsequently disposed.

10. Method according to claim 8 or 9, **characterized in that** at least the ionic compound of the first layer (31) is applied by means of immersion and/or spraying and/or atomization and/or by means of jet-blasting jet particles on during a tribochemical treatment.

11. Method according to claim 10, **characterized in that** the jet particles contain the ionic compound in a micro-encapsulation, where the micro-encapsulation preferably has at least one thermolabile and/or one photolabile planned breaking point and/or a mechanically labile planned breaking point.

12. Method according to claim 10 or 11, **characterized in that** the tribochemical treatment takes place in at least two steps, namely in a first step, tribochemical treatment by means of jet particles that exclusively or at least predominantly contain an inert hard material, and in a second step that follows the first, tribochemical treatment by means of jet particles that contain the ionic compound.

13. Method according to one of claims 10 to 12, **characterized in that** after tribochemical treatment, storage of the tribochemically treated implant body at elevated humidity, particularly at a humidity greater than 80%, and/or at elevated temperature, particularly at a temperature of more than 50°C, takes place.

14. Implant, particularly intraluminal endoprosthesis, having a body that contains metallic material, preferably iron, which can be obtained by means of a method according to one of claims 8 to 13.

## Revendications

1. Implant, notamment endoprothèse intraluminale, doté d'un corps (30) contenant une matière métallique, de préférence, du fer, où le corps d'implant (30) présente sur une partie de sa surface une première couche (31) avec au moins un composé ionique qui contient des ions d'au moins un halogène, **caractérisé en ce qu'**une troisième couche (33), laquelle présente du stéarate de magnésium, est disposée au moins sur une partie de la première couche (31), ou éventuellement, d'une deuxième couche (32), laquelle recouvre au moins partiellement la première couche, la deuxième couche formant une barrière de diffusion pour les ions de l'au moins un halogène du composé ionique.

2. Implant selon la revendication 1, **caractérisé en ce que** le composé ionique contient des ions chlorures et/ou des ions bromures.

3. Implant selon l'une des revendications précédentes, **caractérisé en ce que** le composé ionique est un composé du groupe contenant NaCl, CaCl₂, MgCl₂.

4. Implant selon l'une des revendications précédentes, **caractérisé en ce que** la première couche (31) présente au moins encore un support provenant du groupe contenant des polylactides, des polyglycosides, des copolymères de ces polymères et un phosphate alcalino-terreux.

5. Implant selon l'une des revendications précédentes, **caractérisé en ce que** le composé ionique présente au moins un cation des éléments provenant du groupe contenant les éléments du premier groupe principal, les éléments du deuxième groupe principal, du zinc et de l'arsenic.

6. Implant selon l'une des revendications précédentes, **caractérisé en ce qu'**une troisième couche (33), laquelle présente des parylènes et/ou une substance pharmaceutiquement active, est disposée sur au moins une partie de la première couche (31) ou éventuellement de la deuxième couche (32).

7. Implant selon l'une des revendications précédentes, **caractérisé en ce que** le corps de l'implant présente principalement du fer, de préférence, plus de 80 % en poids, de manière particulièrement préférée, plus de 99 % en poids de fer, notamment dans un alliage.

8. Procédé de fabrication d'un implant, notamment, selon l'une des revendications précédentes, doté d'un corps (30) contenant une matière métallique, de préférence du fer, comprenant les étapes suivantes :
a) de mise au point du corps de l'implant,
b) de dépôt des constituants de la première couche (31) sur au moins une partie de la surface du corps d'implant, où, de préférence, le composé ionique est déposé séparément des autres constituants de la première couche (31)
c) de dépôt d'une troisième couche, qui contient du stéarate de magnésium, sur au moins une partie de la première couche.

9. Procédé selon la revendication 8, **caractérisé en ce qu'**avant le dépôt du premier constituant de la première couche (31), on rapporte un promoteur d'adhésion sur la surface du corps d'implant au moins dans les régions sur lesquelles les constituants de la première couche seront ensuite déposés.

10. Procédé selon la revendication 8 ou 9, **caractérisé en ce qu'**au moins le composé ionique de la première couche (31) et déposé au moyen d'une immersion, et/ou d'une pulvérisation, et/ou d'un saupoudrage, et/ou au moyen d'un dépôt par rayonnement de particules excitées pendant un traitement tribochimique.

11. Procédé selon la revendication 10, **caractérisé en ce que** les particules excitées contiennent le composé ionique dans un micro-encapsulage, où le micro-encapsulage présente de préférence au moins un point de rupture souhaitée thermolabile et/ou photo-labile, et/ou un point de rupture souhaitée labile mécaniquement.

12. Procédé selon la revendication 10 ou 11, **caractérisé en ce que** le traitement tribochimique a lieu en au minimum deux étapes, à savoir, dans une première étape, un traitement tribochimique au moyen de particules excitées qui présentent exclusivement, ou au moins principalement, au moins une matière dure inerte, et dans une deuxième étape consécutive à la première étape, un traitement tribochimique au moyen de particules excitées, lesquelles contiennent le composé ionique.

13. Procédé selon l'une des revendications 10 à 12, **caractérisé en ce qu'**après un traitement tribochimique, il y a un transfert du corps d'implant traité par tribochimie pour une humidité d'air élevée, notamment, pour une humidité de l'air supérieure à 80 %, et/ou pour une température élevée, notamment pour une température supérieure à 50 °C.

14. Implant, notamment endoprothèse intraluminale, dotée d'un corps contenant une matière métallique, de préférence, du fer, pouvant être obtenu par un procédé selon l'une des revendications 8 à 13.
